## Europäisches Patentamt
## European Patent Office
## Office européen des brevets

(19)

(11) Veröffentlichungsnummer: **0 158 285 B1**

# EUROPÄISCHE PATENTSCHRIFT

(12)

(45) Veröffentlichungstag der Patentschrift: 20.03.91

(51) Int. Cl.5 **C12N 5/06**

(21) Anmeldenummer: 85104102.0

(22) Anmeldetag: 04.04.85

(54) Verfahren zur Gewinnung von Zellkulturen.

(30) Priorität: 13.04.84 DE 3413960

(43) Veröffentlichungstag der Anmeldung:
16.10.85 Patentblatt 85/42

(45) Bekanntmachung des Hinweises auf die
Patenterteilung:
20.03.91 Patentblatt 91/12

(84) Benannte Vertragsstaaten:
AT BE CH DE FR GB IT LI LU NL SE

(56) Entgegenhaltungen:

BIOLOGICAL ABSTRACTS, Band 68, Nr. 10,
1979, Seite 6437, Zusammenfassung Nr.
64182, Philadelphia, Penn., US; G. LANG:
"Collagenase in equine cell culture preparation", & J. CLIN. MICROBIOL 9(6), 731-733,
1979

BIOLOGICAL ABSTRACTS, Band 69, Nr. 6,
1979, Seite 4400, Zusammenfassung Nr.
41128, Philadelphia, Penn., US; D.W. SCHO-
LER et al.: "Isolation of rat kidney cortical
tubules enriched in proximal and distal segments", & AM. J. PHYSIOL. 237(5), F350-F359,
1979

(73) Patentinhaber: BEHRINGWERKE Aktiengesellschaft
Postfach 1140
W-3550 Marburg 1(DE)

(72) Erfinder: Bengelsdorff, Hans-Joachim, Dr.
Oberer Eichweg 39
W-3550 Marburg 1(DE)
Erfinder: Koschel, Eckhard
Friedrich-Ebert-Strasse 54
W-3578 Schwalmstadt 1(DE)

(74) Vertreter: Beck, Bernhard et al
HOECHST AKTIENGESELLSCHAFT Zentrale
Patentabteilung Postfach 80 03 20
W-6230 Frankfurt/Main 80(DE)

BIOLOGICAL ABSTRACTS, Band 74, Nr. 2, 1979, Zusammenfassung Nr. 8677, Philadelphia, Penn. US; D. DEUTSCH et al.: "Purification and further characterization of isolated matrix vesicles from rat alveolar bone", & METAB. BONE DIS. RELAT. RES. 3(3), 209-214, 1981

METHODS IN ENZYMOLOGY, Band LVIII, 1979, Seiten 119-131, Academic Press Inc.; M.M. BASHOR: "Dispersion and disruption of tissues"

## Beschreibung

Herstellungsverfahren für Zellkulturen und auch für solche aus Schweinenieren zur Gewinnung von Virussuspensionen als Vaccineantigene sind bekannt. Sie basieren zum Beispiel auf der Verwendung von Schweineembryonieren, Ferkelnieren, permanenten Schweinenierenzellstämmen oder Schlachtschweinnieren.

Nachteile der Verfahren des Standes der Technik für die industrielle Produktion sind: Schweineembryonieren sind für industrielle Produktionsmaßstäbe nicht in genügender Menge und zu den gewünschten Zeiten erhältlich. Ferkelnieren sind relativ klein, das heißt sie erbringen bei zwar gutem Zellwachstum nur eine geringe Zellausbeute. Außerdem ist der zugehörige Tierkörper nach der Nierenentnahme als Nahrungsmittel in der Regel unbrauchbar. Permanente Schweinenierenzellstämme wie PK 15, IB S 2, STA oder SK 6 sind zwar in großen Mengen vermehrbar, sind aber häufig mit Fremdagenzien wie Mycoplasmen oder Viren kontaminiert, so daß ihre industrielle Nutzung für die Herstellung von immunbiologischen Produkten ein erhöhtes Risiko beinhaltet. Auch ist der mit der Zellstammhaltung verbundene Aufwand einschließlich der laufenden qualitativen Überprüfungen beträchtlich. Hinzu kommt noch, daß sich viele Viren auf permanenten Zellstämmen wegen deren genetischer Veränderungen nicht oder im Vergleich zu primären Zellkulturen aus Organzellen nur schlecht vermehren.

Schlachtschweinnieren sind eine kostengünstige Zellbasis. Nieren ausgewachsener Schlachtschweine erbringen jedoch im Vergleich zu embryonalen oder Ferkelnieren nur relativ wenige Zellen mit Zellteilungsaktivtät. Wenn nun Schlachtschweinnieren noch auf herkömmliche Weise mit dem zellschädigenden Verdauungsferment Trypsin aufgeschlossen werden, so reduziert sich die Anzahl vitaler Zellen in einer so angelegten Zellkultur nochmals drastisch. Dadurch wird verständlich, daß auf herkömmliche Weise fermentativ aufgeschlossene Suspensionen aus Nierenzellen von Schlachtschweinen nur mit erheblichem Aufwand wie durch Mediumwechsel, erhöhten Serumzusatz zum Kulturmedium oder spezielle Kultivierungsmedien und nach langer Kultivierungsdauer zu meist nur partiell geschlossenen Zellrasen führen.

Zellkulturen aus Schlachtschweinnieren, hergestellt durch den üblichen Trypsinaufschluß der Organe, sind also problematisch im Hinblick auf ihre Wuchsfreudigkeit. Insbesondere treten diese Wachstumsschwierigkeiten dann hervor, wenn die Kulturgefäße mit den tryptisch aufgeschlossenen Schlachtschweinnierenzellen zur optimalen Ausnutzung der Kultivierungsfläche gerollt werden.

Es bestand deshalb weiterhin ein Bedarf an einem auch technisch befriedigenden Verfahren zur Gewinnung von Zellkulturen aus Nierenzellen von Schweinen.

Es wurde nun überraschenderweise ein Verfahren gefunden, das es gestattet, Nierenzellen von Schlachtschweinen ohne besondere Mediumzusätze und ohne Mediumwechsel in 5 bis 8 Tagen in stationären oder Rollkulturen zu dichten Zellrasen auswachsen zu lassen. Dieses Verfahren ist dadurch gekennzeichnet, daß zum Aufschluß der Nieren zu Einzelzellen Collagenaselösung benutzt wird.

Gegenstand der Erfindung ist deshalb ein Verfahren zur Gewinnung einer Zellkultur durch Aufschluß von Nieren von Schlachtschweinen mittels eines Enzyms, dadurch gekennzeichnet, daß das Enzym Collagenase ist.

Eine verwendbare Collagenase kann beispielsweise gewonnen werden aus Clostridium histolyticum und wird in einer Konzentration von 10-15, vorzugsweise 12,5 mg (entsprechend 6.250 Mandl-Einheiten) pro 100 ml phosphatgepufferter Salzlösung (PBS, pH-Wert 7,0 bis 7,5) eingesetzt.

Von besonderer Bedeutung für die Gewinnung großer Mengen an vitalen, mitoseaktiven Einzelzellen ist eine unter Rühren 12- bis 24-stündige Einwirkungsdauer der Collagenaselösung bei 16 bis 22°C auf die zerkleinerten Nieren.

Die so gewonnenen Einzelzellen werden dann in bekannter Weise durch Zentrifugation bei etwa 800 x g und Waschen mit PBS gereinigt. Das zentrifugierte Zellsediment wird danach im Verhältnis von vorzugsweise 1:250 bis 1:350 in bekannten Kultivierungsmedien wie Eagles minimal essential medium (Eagles MEM) oder in TCM 199 mit Zusatz von 100 ml/l Kälberserum resuspendiert, in Kulturgefäße portioniert, die bei 37°C stationär gehalten oder gerollt werden. Innerhalb von 8 bis 16 Stunden beginnen zahlreiche der an der Glasfläche sedimentierten Nierenzellen sich zu teilen. Dieser Vorgang kann mikroskopisch beobachtet werden und zeigt, daß ein signifikanter Unterschied in der Anzahl der teilungsfähigen Nierenzellen und in der Schnelligkeit der Zellteilungsfolgen im Vergleich zu Zellansätzen nach herkömmlichen Kultivierungsverfahren besteht. Die Mitoseaktivität der mit Collagenase behandelten Nierenzellen von Schweinen führt zu schnellem Dichtwachsen des Zellrasens ohne irgendwelche weiteren Hilfsmaßnahmen für die Zellkultur. Hierdurch können - wie nachstehende Beispiele erläutern - in rascher Folge große Mengen an Zellkulturen für die industrielle Viruszüchtung vorteilhaft hergestellt werden.

Beispiel 1

Schlachtschweinnieren wurden nach den herkömmlichen Verfahren mit 2 g/l Trypsinlösung zu Einzelzellen aufgeschlossen, nach Zentrifugation bei etwa 800 g mit PBS gewaschen und erneut zentrifugiert. Das zellsediment wurde im Verhältnis 1:300 in Eagles MEM suspendiert, dem 2,5 g/l Laktalbuminhydrolysat (LAH) und 100 ml/l Kälberserum zugesetzt worden waren. Diese Suspension wurde in Mengen von 600 ml in sterile 5 1-Glaskolben gefüllt. Die Kulturen wurden unter Rollen bei 37°C bebrütet. Innerhalb von 10 bis 14 Tagen hatten sich nur partiell geschlossene Zellrasen gebildet. Nach Wechseln des Wachstumsmedium auf serumfreies Medium (Eagles MEM mit 2,5 g/ l LAH) wurden die Kulturen mit Reovirus der Serotypen 1 bzw. 3 infiziert und bis zur Erntereife der Kultur 3 bis 5 Tage bei 37°C gehalten. Der Virusgehalt der Kulturabgüsse wurde nach Entfernung der Zellreste mittels Zentrifugation und anschließender Filtration in üblicher Weise durch Verimpfung von Verdünnungsstufen auf Zellkulturen geprüft.

Zum Vergleich wurden steril zerkleinerte Schlachtschweinnieren mit Collagenaselösung, die 12 5 mg Collagenase pro 1 enthielt (entsprechend 6.250 Mandl-Einheiten/100 ml), behandelt. Nach 18-stündiger Einwirkungsdauer bei 18°C wurden die zu Einzelzellen aufgeschlossenen Nierenzellen in gleicher Weise wie oben weiterverarbeitet. Die Kulturmedien, die Kulturgefäße, die abgefüllten Zellsuspensionsmengen und die Bebrütung waren gleich. Im Gegensatz zu den Zellkulturen nach dem herkömmlichen Verfahren waren bei den nach dem erfindungsgemäßen Verfahren aufgeschlossenen Nierenzellen bereits nach wenigen Stunden Mitosen mikroskopisch erkennbar. Auch lagen die einzelnen mitoseaktiven Zellen sehr eng beieinander, da die Anzahl der geschädigten und deshalb amitotischen Zellen gegenüber dem herkömmlichen Aufschlußverfahren stark reduziert war. Aufgrund der sehr früh beginnenden und sehr zahlreichen Mitosevorgänge waren 5 bis längstens 8 Tage nach Zellkulturansatz dicht geschlossene infektionsfähige Gewebekulturen vorhanden. Die Infektion der Gewebekulturen sowie die Virusernte und -prüfung erfolgten in analoger Weise wie bei den nach dem herkömmlichen Verfahren hergestellten Kulturen.

Nach den beiden beschriebenen Verfahren wurden je 5 Kulturansätze im technischen Maßstab hergestellt, auf denen jeweils 150 bis 300 1 Reovirus der Serotypen 1 und 3 gezüchtet wurden. Die Virusgehalte der Vergleichsansätze wurden auf permanenten Affennierenzellen (Verozellen) bestimmt. Für Reovirus des Serotyps 3 wurden bis 14,5-fach und für den Serotyp 1 wurden 5,6- bis 6,3-fach höhere Titer gefunden, wenn mit dem erfindungsgemäßen Verfahren anstelle des Verfahrens nach dem Stand der Technik gearbeitet wurde.

de.

### Beispiel 2

Für die Herstellung von 40 1 einer Suspension von Rhinopneumonitis -Virus (Erreger des Stutenabortes) auf Ferkelnierenzellen wurden 20 junge Ferkel benötigt, während nach dem erfindungsgemäßen Verfahren des Beispiels 1 nur 2 Nierenpaare von Schlachtschweinen gebraucht werden. Ein qualitativer Nachteil des Virus, hergestellt nach dem erfindungsgemäßen Verfahren, wurde nicht beobachtet.

### Beispiel 3

Das Aujeszky-Virus ist als Herpesvirus ein schlechtes Antigen, deshalb kommt einem hohen Virusgehalt in der Vaccine eine große Bedeutung zu. Es wurden vergleichend Aujeszky-Viruszüchtungen auf Nierenkulturen von Schlachtschweinen durchgeführt, die einmal nach dem herkömmlichen Verfahren mit trypsinaufgeschlossenen Nierenzellen und zum anderen nach dem erfindungsgemäßen Verfahren (Beispiel 1) hergestellt worden waren. Während der Virustiter/ml von den nach dem herkömmlichen Verfahren hergestellten Kulturen bei $10^{-7,5}$ lag, betrug er bei den nach dem erfindungsgemäßen Verfahren hergestellten Kulturen im Mittel $10^{-8,5}$, das heißt es wurde von den jüngeren (5-8 Tage alten) und dichten Gewebekulturen etwa zehnmal mehr Virus synthetisiert als von den älteren (10-14 Tage alten) und in der Regel nicht dichten Kulturen des Standes der Technik.

### Beispiel 4

Schweineparvovirus (PPV) gilt als Erreger des SMEDI-Syndroms bei Zuchtsauen, das durch Aborte, Totgeburten, Geburt lebensschwacher Ferkel und Sterilität bei Sauen charakterisiert ist. Das PPV vermehrt sich nur in jungen, noch wachsenden Gewebekulturen von Schweinen, vorzugsweise in Schweinenierenkulturen. Versuche zur Züchtung von PPV in Gewebekulturen von Schlachtschweinen, hergestellt nach dem erfindungsgemäßen Verfahren, haben ergeben, daß solche Zellkulturen besonders gut für die Vermehrung von diesem Virus geeignet sind. Es wurden Haemagglutinationstiter von 1:2048 bis 1:16.384 gefunden, während Virustiter von 1:1024 für nach dem Stand der Technik in embryonalen Schweinenierenzellkulturen oder permanenten Schweinenierenzellstämmen vermehrtes PPV schon als gut gelten.

## Ansprüche

Verfahren zur Gewinnung einer Zellkultur durch Aufschluß der Nieren von Schlachtschweinen mittels eines Enzyms, dadurch gekennzeichnet, daß man eine Collagenaselösung mit einer Konzentration von 100-150 mg/l 12 bis 24 Stunden bei 16 bis 22°C auf die zerkleinerten Nieren unter Rühren einwirken läßt und die so gewonnenen Schweinenierenzellen in bekannter weise zu Zellkulturen auswachsen läßt.

## Claims

A process for obtaining a cell culture by disintegration of the kidneys of slaughtered pigs using an enzyme, which comprises exposing the comminuted kidneys to a stirred collagenase solution with a concentration of 100-150 mg/l for 12 to 24 hours at 16 to 22°C and allowing the pig kidney cells obtained in this way to grow to cell cultures in a known manner.

## Revendications

Procédé d'obtention de cultures de cellules, par désagrégation de reins de porc d'abattage au moyen d'une enzyme, caractérisé en ce que l'on fait agir une solution de collagénase ayant une concentration de 100 à 150 mg/l pendant 12 à 24 heures à une température de 16°C à 22°C sur des reins coupés en petits morceaux, sous agitation, et que l'on cultive les cellules de reins de porcs ainsi isolées, de manière connue en cultures cellulaires.